Europäisches Patentamt

European Patent Office

Office européen des brevets

(11) Veröffentlichungsnummer : **0 111 942**
**B1**

(12) **EUROPÄISCHE PATENTSCHRIFT**

(45) Veröffentlichungstag der Patentschrift :
02.03.88

(51) Int. Cl.⁴ : **G 01 N 33/49**

(21) Anmeldenummer : **83113040.6**

(22) Anmeldetag : **23.12.83**

(54) Einrichtung zur Messung der Blutungszeit in vitro.

(30) Priorität : **23.12.82 DE 3247815**

(43) Veröffentlichungstag der Anmeldung :
**27.06.84 Patentblatt 84/26**

(45) Bekanntmachung des Hinweises auf die Patenterteilung : **02.03.88 Patentblatt 88/09**

(84) Benannte Vertragsstaaten :
**AT BE CH DE FR GB IT LI LU NL SE**

(56) Entgegenhaltungen :
**GB-A- 1 263 894**
**GB-A- 1 469 151**
**GB-A- 2 096 329**

(73) Patentinhaber : **Kratzer, Michael, Dr.**
**Leopoldstrasse 56**
**D-8000 München 40 (DE)**

**Born, Gustav Viktor Rudolf, Prof.**
**10 Woodland Gardens Muswell Hill**
**London N10 3UA (GB)**

(72) Erfinder : **Kratzer, Michael, Dr.**
**Leopoldstrasse 56**
**D-8000 München 40 (DE)**
Erfinder : **Born, Gustav Viktor Rudolf, Prof.**
**10 Woodland Gardens Muswell Hill**
**London N10 3UA (GB)**

(74) Vertreter : **Von Puttkamer, Nikolaus**
**Pienzenauerstrasse 2**
**D-8000 München 80 (DE)**

## Beschreibung

Die vorliegende Erfindung betrifft eine Einrichtung zur Messung der Blutungszeit in vitro, wobei bei der Einrichtung das Blut unter Druck durch ein poröses Teil beförderbar ist und wobei eine Einrichtung zur direkten oder indirekten Messung des resultierenden Volumenstromes vorgesehen ist, und ein Verfahren zur Messung der Blutungszeit in vitro.

Im Blutplättchen-Aggregometer nach G.V.R. Born wird bekannterweise plättchenreiches Plasma mit einem Rührwerk ständig in konvektiver Bewegung gehalten. Die Aggregation der Plättchen wird durch einen definierten Reiz, beispielsweise durch die Zugabe von Adenosindiphosphat ausgelöst. Gleichzeitig wird mit Hilfe einer Photozelle die Lichttransmission gemessen. Wenn die Blutzellen aggregieren, so nimmt die Lichttransmission zu. Die Amplitude und die Geschwindigkeit der Zunahme des Photostromes stellen ein Maß für die Aggregationsneigung der Blutplättchen dar.

Bei einem weiteren von Didisheim beschriebenen Verfahren für die Bestimmung der in vitro Blutungszeit wird natives oder anticoaguliertes Blut durch einen Plastikschlauch gepumpt, in dem sich seitenständig eine Öffnung mit einem Innendurchmesser von etwa 200 μm befindet. Diese Öffnung wird nach etwa 5 min durch das ausfließende Blut verstopft. Dabei ist die durch diese Einrichtung ermittelte Blutungszeit u. a. abhängig von der Aggregationsneigung der Blutplättchen.

Aus der GB-PS 2 096 329 sind ein Verfahren und eine Einrichtung bekannt, durch die die Aggregationsneigung von im Blut suspendierten Thrombozyten meßbar ist. Dabei wird eine Blutprobe in einem Behälter auf einer im wesentlichen konstanten Temperatur gehalten. In diesen Behälter taucht das Ende eines Röhrchens ein, dessen anderes Ende mit einer einen konstanten Unterdruck erzeugenden Quelle über ein Ventil verbunden ist. Beim Öffnen des Ventiles wird bewirkt, daß das Blut durch das eine Ende des Röhrchens gesaugt wird. Der Durchmesser des Röhrchens ist im Bereich des einen Endes so bemessen, daß die gebildete Apertur während des Experimentes durch die Aggregation der im Blut suspendierten Thrombozyten verschlossen wird. Durch eine Meßeinrichtung wird der sich unter konstantem Druckgefälle ergebende Volumenstrom des Blutes durch die Apertur gemessen. Auf diese Weise ist es möglich, den Vorgang der Blutung in vitro zu simulieren.

Die Aufgabe der vorliegenden Erfindung besteht darin, eine Einrichtung und ein Verfahren zur Messung der Blutungszeit in vitro der eingangs genannten Art dahingehend zu verbessern, daß die Blutungsverhältnisse unter in vivo-Bedingungen genauer nachgebildet werden können, als dies mit den bekannten vergleichbaren Einrichtungen bzw. Verfahren möglich ist.

Diese Aufgabe wird durch eine Einrichtung der eingangs genannten Art, die dadurch gekennzeichnet ist, daß im porösen Teil mindestens eine Apertur vorgesehen ist, daß das poröse Teil auf einer im Bereich der Apertur eine Öffnung aufweisenden luftundurchlässigen Auflage aufliegt und daß das Blut derart zuführbar ist, daß es durch die Apertur hindurchtreten und nicht seitlich an dem porösen Teil vorbeigelangen kann und durch ein Verfahren der eingangs genannten Art gelöst, das dadurch gekennzeichnet ist, daß das Material des porösen Teiles vor Beginn einer Untersuchung mit einer die Blutplättchen aggregierenden Lösung getränkt wird.

Einwesentlicher Vorteil der vorliegenden Erfindung besteht darin, daß die Störanfälligkeit des mit ihr durchgeführten Meßverfahrens durch Mikrothromben im Blut wesentlich herabgesetzt und die Reproduzierbarkeit wesentlich erhöht werden kann.

Ein weiterer Vorteil der Erfindung besteht darin, daß das mit der vorliegenden Einrichtung durchgeführte Verfahren in wesentlichen Grundzügen der in vivo Situation der Blutung (aus einer kleinen Arterie) nachgebildet ist.

Gemäß dem vorliegenden Verfahren wird das poröse Teil mit einer die Plättchen aggregierenden Lösung, beispielsweise einer Adenosindiphosphatlösung, getränkt. Dadurch ist es vorteilhafterweise möglich, die Blutungsverhältnisse unter in vivo Bedingungen noch genauer nachzuahmen, weil bei einer tatsächlichen Verletzung aus der verletzten Gefäßwand Adenosindiphosphat freigesetzt wird, wodurch die Plättchenaggregation induziert wird.

Gemäß einer anderen Ausgestaltung der vorliegenden Erfindung ist der Apertur eine Kapillare vor- oder nachgeschaltet. Dadurch ist es vorteilhafterweise möglich, den Widerstand einer kleinen Arterie, aus der die Blutung stattfindet, zu simulieren, und haemodynamische Verhältnisse unter in vivo Bedingungen nachzuahmen.

Die Aggregationsneigung von Blutplättchen, die unter anderem die Blutungszeit bestimmt, ist ein in der Klinik häufig zur Diagnose und für die Beurteilung der Medikamentenwirksamkeit von Antiaggregationssubstanzen verwendeter Parameter. Dabei werden derartige Substanzen beispielsweise bei Herzinfarkten oder arterielleren Verschlüssen verabreicht. Durch die Erfindung können derartige Diagnosen bzw. Beurteilungen vorteilhafterweise erstmalig einfach, schnell und reproduzierbar ohne einen großen Personalaufwand durchgeführt werden.

Im folgenden wird die vorliegende Erfindung im Zusammenhang mit den Figuren näher erläutert. Es zeigt:

Fig. 1 eine Einrichtung zur Messung der Blutungszeit unter in vitro Bedingungen, und

Fig. 2 in vergrößerter Darstellung das aus porösem Material bestehende Teil, das die Apertur aufweist, und

Fig. 3 eine Ausgestaltung der erfindungsgemäßen Einrichtung.

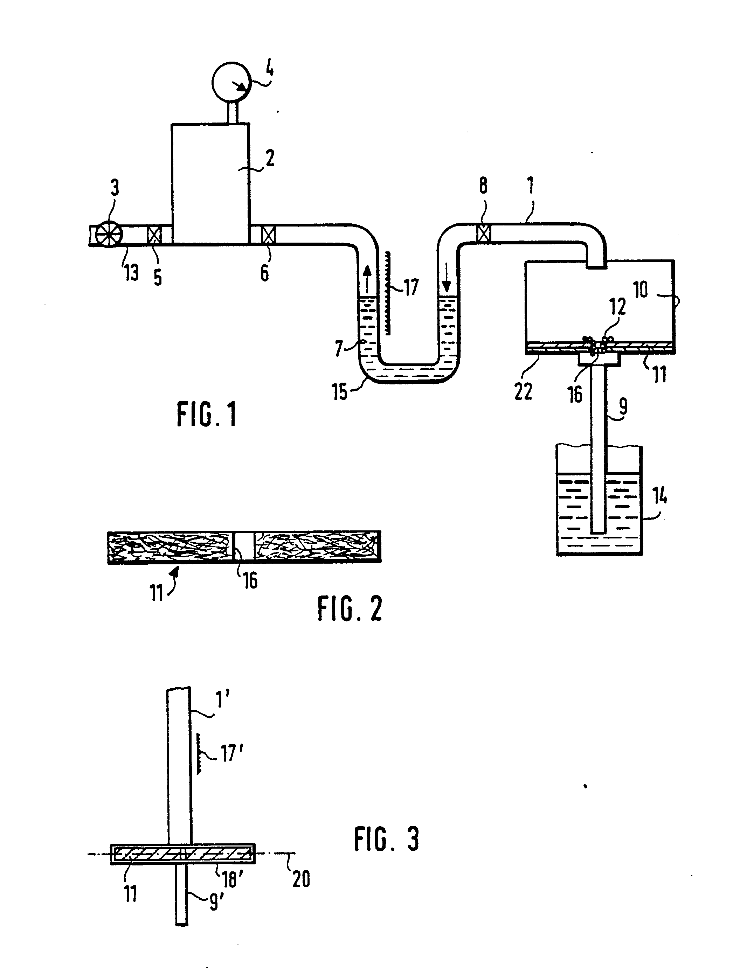

In der Fig. 1 ist ein Element, das ein Teil 11 aus porösem Material aufnimmt und hält mit 10 bezeichnet. Mit dem Element 10 ist ein Rohr 1 dicht verbunden, das mit einem Reservoir 2 mit einem negativen Druck in Verbindung steht. Dabei wird der negative Druck in dem Reservoir 2 durch eine Pumpe 3 erzeugt, die über eine Leitung 13 und ein Ventil 5 mit dem Reservoir 2 verbunden ist. An dem Reservoir 2 ist eine Meßeinrichtung 4 vorgesehen, durch die der in dem Reservoir 2 herrschende negative Druck anzeigbar ist.

Das Blut, das über eine Kapillare 9 durch die Apertur 16 des Teiles 11 aus porösem Material, das im folgenden noch näher beschrieben werden wird, gesaugt werden soll, befindet sich in einem Behälter 14, bei dem es sich beispielsweise um ein Kunststoffnäpfchen handeln kann, das, wie dies in der GB-PS 2 096 329 beschrieben ist, in einem Metallgefäß angeordnet sein kann, in dem sich Wasser befindet, das auf einer Temperatur von 37 °C gehalten wird.

Um zu verhindern, daß Luft durch das poröse Material gesaugt wird, liegt das Teil 11 auf einer Auflage 22 auf, die im Bereich der Apertur 16 eine Öffnung aufweist. Die Auflage 22 ist dicht mit dem Element 10 und der Kapillare 9 verbunden. Beispielsweise besteht die Auflage 22 aus einer Platte und einer darauf aufgebrachten Gummischicht, die sich zwischen der Platte und dem porösen Material befindet. Außerdem wird durch die Auflage sichergestellt, daß kein Blut seitlich entlang der Außenflächen von einer Seite des Teiles 11 zur anderen Seite des Teiles 11 fließt. Dies kann beispielsweise auch dadurch erreicht werden, daß das Teil 11 zwischen zwei Platten zusammengepreßt wird, die im Bereich der Apertur 16 Öffnungen aufweisen und dort dicht mit der Kapillare 9, 9' bzw. dem Rohr 1 verbunden sind.

Vorzugsweise ist in dem Rohr 1 ein U-förmiger Bereich 15 vorgesehen, in dessen Inneren sich eine gefärbte Flüssigkeit 7 befindet. Wenn beim Öffnen der Ventile 6 und 8 Blut durch die Apertur 16 des Teiles 11 gesaugt wird, wird die in dem U-förmigen Bereich 15 befindliche gefärbte Flüssigkeit 7 in die durch die Pfeile angedeutete Richtung verschoben.

Dabei ist die Verschiebung der gefärbten Flüssigkeit 7 ein Maß für den sich durch die Apertur 16 ergebenden Volumenstrom des Blutes. Die Verschiebung des Pegels der gefärbten Flüssigkeit 7 in einem Schenkel des U-förmigen Bereiches 15 kann durch eine schematisch dargestellte Meßeinrichtung 17 ermittelt werden. Vorzugsweise handelt es sich bei dieser Meßeinrichtung 17 um eine elektrooptische Meßeinrichtung der in der GB-PS 2 096 329 (Fig. 2) beschriebenen Art, die mit einem Recorder verbunden sein kann, der die Änderungen des Volumenstromes des Blutes durch die Apertur 16 in Abhängigkeit von der Zeit aufzeichnet.

An der Stelle des Rohres 1 mit dem U-förmigen Bereich 15 kann zur Ermittlung des Volumenstromes durch die Apertur 16 auch die Verschiebung einer gefärbten Flüssigkeit in einem geradlinigen Rohr gemessen werden, das horizontal angeordnet ist. Eine derartige Meßeinrichtung ist ebenfalls in der GB-PS 2 096 329 (Fig. 3) beschrieben.

Es ist außerdem auch möglich, die zwischen Stellen vor und hinter der Apertur bestehende Druckdifferenz zu messen. Diese Messung stellt ein charakteristisches Maß für den Grad der Verschließung der Apertur, d. h. für die Aggregation des Blutes im Bereich der Apertur dar.

Das poröse Material des Teiles 11 ist so beschaffen, daß die Blutpartikel nicht durch das Material hindurchgesaugt werden können. Dadurch wird erreicht, daß die Blutpartikel tatsächlich nur durch die Apertur hindurchtreten und beispielsweise nicht in die Randbereiche der Apertur 16 eintreten.

Das Teil 11 aus porösem Material besteht vorzugsweise aus einem Filtermaterial, in das ein einziges Loch, das die Apertur 16 bildet, gestanzt ist. In der Fig. 2 ist das Teil 11 vergrößert dargestellt.

Vorzugsweise weist das Filtermaterial eine Porengröße auf, die kleiner als 5 μm und größer als 0,01 μm ist. Beispielsweise weist das Filtermaterial die Form eines Filters der Firma Millipore auf, der unter der Bezeichnung GSWP 01300 bezogen werden kann und dessen Poren einen Durchmesser von 0,22 μm aufweisen. Der Durchmesser der in dem Teil 11 vorgesehenen Apertur 16 liegt vorzugsweise in einem Bereich von 50 μm bis 300 oder 500 μm. Insbesondere beträgt der Durchmesser der Apertur 16 150 μm bis 250 μm, beispielsweise 200 μm.

Das Teil 11 weist vorzugsweise die Form einer Scheibe oder eines länglichen Röhrchens auf.

Vorzugsweise wird das Filtermaterial mit einem die Plättchen des Blutes aggregierenden Material getränkt. Die Erfinder haben nämlich herausgefunden, daß die haemostatische Aggregation der Blutplättchen nach einer Gefäßverletzung durch Nucleotide eingeleitet wird, die schnell von den beschädigten Zellen freigegeben werden. Dieser Vorgang kann dadurch simuliert werden, daß das poröse Material bzw. das Filtermaterial mit dem die Plättchen aggregierenden Material getränkt wird, bei dem es sich beispielswiese um eine Adenosindiphosphatlösung handelt. Die Lösung gelangt dann über die Poren des Filtermaterials zur Apertur 16 und in den durch die Apertur 16 fließenden Blutstrom.

Nach dem Öffnen der Ventile 6 und 8 wird das beispielsweise auf 37 °C erwärmte antikoagulierte menschliche Blut (beispielsweise eine Mischung : Na-Citrat/Blut = 1:9) durch die Kapillare 9, deren Innendurchmesser beispielsweise 140 μm beträgt und deren Länge beispielsweise 1,6 cm beträgt, und durch die Apertur 16 gesaugt. Beispielsweise beträgt die dabei durch das Reservoir 2 und die Pumpe 3 erzeugte negative Druckdifferenz 5 000 Pa. Wenn das Blut durch die Apertur 16 fließt, wird diese allmählich durch Blutplättchen 12 verschlossen, die einen Pfropfen bilden. Der Blutfluß wird daher allmählich verlangsamt und schließlich völlig unterbrochen. Durch eine Bemessung des Innendurchmessers der Kapillare 9, die der Apertur 16 auch nachgeschaltet sein kann, ist es vorteilhafterweise möglich, den Strömungswiderstand einer kleinen Arterie, aus der die Blutung stattfindet, genau zu simulieren. Auf diese Weise können haemodynamische Verhältnisse unter in vivo Bedingungen nachgeahmt werden.

Im folgenden wird ein Beispiel für eine mit der vorliegenden Einrichtung durchgeführte Messung angegeben.

Das Blut eines gesunden Spenders (Mittelwert : SEM (Standard Error of the Mean) n = 8) wurde durch die Apertur gesaugt. Der der Viskosität des Blutes proportionale Blutfluß betrug nach dem Beginn der Untersuchung 6 7 + 2,8 $\mu$l/min. Er endete nach einer « Blutungszeit » von 2,9 $\pm$ 0,6 min durch die Ausbildung eines Thrombus. Während der Untersuchung flossen insgesamt 186 $\pm$ 19 $\mu$l Blut durch die Apertur 16, die einen Durchmesser von 150 $\mu$m aufwies. Eine Untersuchung des Filtermaterials 11 unter dem Elektronenmikroskop zeigt die Abscheidung eines aus Blutplättchen bestehenden Pfropfens. Wenn das Filtermaterial mit einer die Plättchenthrombusbildung hemmenden Adenosinlösung ($10^{-2}$ Mol/Liter) getränkt war, bildete sich kein Thrombus aus.

Um eine noch bessere Reproduzierbarkeit und kürzere Blutungszeiten erreichen zu können, kann das Filtermaterial des Teiles 11 mit einem säurelöslichen Kollagen durchsetzt werden. Dies hat den Vorteil, daß im Randbereich der Apertur 16 die mit Kollagen beschichteten Filterfasern das Anhaften der Blutplättchen begünstigen.

Es ist auch denkbar, auf die Oberfläche des porösen Materials bzw. des Filtermaterials in einer Kultureinrichtung lebende Zellen auf wachsen zu lassen. Dadurch ist eine noch bessere in vivo Nachbildung möglich.

Fig. 3 zeigt eine besonders einfache Ausgestaltung der Erfindung, bei der der Anstieg des Blutes in einem Röhrchen 1' gemessen wird, das vorzugsweise horizontal oder vertikal angeordnet sein kann und dessen eines Ende mit dem Reservoir 2 verbunden ist.

Am anderen Ende des Röhrchens 1' ist in einer Ausbuchtung das Teil 11 angeordnet, wobei der Boden der Ausbuchtung die Auflage 18' bildet. Unterhalb der Öffnung der Auflage 18' ist die Kapillare 9' befestigt. Zur Ablesung des Pegels des Blutes in dem Röhrchen 1' ist an dem Röhrchen 1' eine Skala 17' vorgesehen. Vorzugsweise ist diese Einrichtung als billiges Wegwerfgerät konzipiert und besteht aus einem Kunststoffmaterial, das nach dem Einbringen des Teiles 11 im Bereich der Ausbuchtung beispielsweise entlang der Linie 20 dicht zusammengesetzt wird. Eine derartige Einrichtung eignet sich hauptsächlich zur Messung einer in einer vorgegebenen Zeit ausgeflossenen Blutmenge.

Für Spezialuntersuchungen können auch mehrere Aperturen in dem Teil 11 vorgesehen werden.

Bei den vorangehenden beschriebenen Ausführungsformen der Erfindung wurde das Blut durch einen negativen Druck des Reservoirs 2 aus einem Behälter durch die Apertur 16 des Teiles 11 gesaugt. Es ist auch denkbar, das Blut in einem Behälter, der dicht mit der Kapillare 9, 9' verbunden wird, unter Druck zu setzen, so daß es in die Kapillare 9, 9' und durch die Apertur 16 gedrückt wird.

## Patentansprüche

1. Einrichtung zur Messung der Blutungszeit in vitro, wobei das Blut unter Druck durch ein poröses Teil beförderbar ist und wobei eine Einrichtung zur direkten oder indirekten Messung des resultierenden Volumenstromes des Blutes vorgesehen ist, dadurch gekennzeichnet, daß im porösen Teil (11) mindestens eine Apertur (16) vorgesehen ist, daß das poröse Teil (11) auf einer im Bereich der Apertur (16) eine Öffnung aufweisenden luftundurchlässigen Auflage (22, 18') aufliegt und daß das Blut derart zuführbar ist, daß es durch die Apertur (16) hindurchtreten und nicht seitlich an dem porösen Teil (11) vorbeigelangen kann.

2. Einrichtung nach Anspruch 1, dadurch gekennzeichnet, daß der Durchmesser der Apertur (16) nicht größer als 500 $\mu$m ist.

3. Einrichtung nach Anspruch 2, dadurch gekennzeichnet, daß der Durchmesser der Apertur (16) nicht größer als 300 $\mu$m ist.

4. Einrichtung nach einem der Ansprüche 1 bis 3, dadurch gekennzeichnet, daß der Durchmesser der Apertur nicht kleiner als 50 $\mu$m ist.

5. Einrichtung nach einem der Ansprüche 1 bis 4, dadurch gekennzeichnet, daß der Durchmesser der Apertur (16) zwischen 150 und 250 $\mu$m liegt.

6. Einrichtung nach einem der Ansprüche 1 bis 5, dadurch gekennzeichnet, daß das poröse Teil (11) aus einem Filtermaterial besteht, das eine Porengröße aufweist, die kleiner als 5 $\mu$m und größer als 0,01 $\mu$m ist.

7. Einrichtung nach einem der Ansprüche 1 bis 6, dadurch gekennzeichnet, daß in dem porösen Teil (11) mehrere Aperturen vorgesehen sind.

8. Einrichtung nach einem der Ansprüche 1 bis 7, dadurch gekennzeichnet, daß der Apertur (16) eine Kapillare (9) vor- oder nachgeschaltet ist und daß die Kapillare einen Innendurchmesser aufweist, der zwischen 50 und 500 $\mu$m liegt.

9. Einrichtung nach Anspruch 8, dadurch gekennzeichnet, daß die Länge der Kapillare (9) 0,5 bis 5 cm beträgt.

10. Einrichtung nach einem der Ansprüche 1 bis 9, dadurch gekennzeichnet, daß das poröse Teil (11) die Form einer Scheibe oder eines länglichen Rohres aufweist.

11. Einrichtung nach einem der Ansprüche 1 bis 10, dadurch gekennzeichnet, daß das poröse Teil (11) aus einem Zellulose-Azetat-Material besteht.

12. Einrichtung nach einem der Ansprüche 5 bis 11, dadurch gekennzeichnet, daß sie als Wegwerfeinrichtung ausgebildet ist und im wesentlichen aus einem eine Skala (17') aufweisenden Röhrchen (1') besteht, das in einem Endbereich eine das poröse Teil (11) aufnehmende Ausbuchtung aufweist, daß die Kapillare (9') an der dem Röhrchen (1') gegenüberliegenden Seite der Ausbuchtung im Bereich der Apertur (16) des porösen Teiles (11) mit der Ausbuchtung verbunden ist, daß das Röhrchen (1') die Ausbuchtung und die Kapillare (9') aus einem Kunststoffmaterial gebildet und nach dem Einsetzen des porösen Teiles (11) in die Ausbuchtung im Bereich der Ausbuchtung dicht und fest zusammengefügt sind.

13. Verfahren zur Messung der Blutungszeit in vitro, mit einer Einrichtung nach einem der Ansprüche 1 bis 12, dadurch gekennzeichnet, daß das Material des porösen Teiles (11) vor Beginn einer Untersuchung mit einer die Blutplättchen (12) aggregierenden Lösung getränkt wird.

14. Verfahren nach Anspruch 13, dadurch gekennzeichnet, daß als Lösung Adenosindiphosphat verwendet wird.

15. Verfahren nach einem der Ansprüche 13 oder 14, dadurch gekennzeichnet, daß das Material des porösen Teiles (11) mit Kollagen durchsetzt wird.

16. Verfahren nach einem der Ansprüche 13 bis 15, dadurch gekennzeichnet, daß auf die Oberfläche des porösen Teiles (11) wenigstens im Bereich der Apertur (16) in einer Kultureinrichtung vor Beginn einer Untersuchung biologische, lebende Zellen aufgewachsen werden.

17. Verfahren nach einem der Ansprüche 13 bis 16, dadurch gekennzeichnet, daß zur Messung des resultierenden Volumenstromes des Blutes die durch einen positiven oder negativen Druck bewirkte Verschiebung einer Flüssigkeitssäule (7) in einem Rohr (1) gemessen wird.

18. Verfahren nach einem der Ansprüche 13 bis 16, dadurch gekennzeichnet, daß zur Messung des Grades der Verstopfung der Apertur (16) die Differenz der unmittelbar an beiden Seiten der Apertur (16) herrschenden Drücke gemessen wird.

**Claims**

1. Assembly for measuring bleeding time in vitro, with the blood being movable under pressure through a porous member, whereby means being provided for directly or indirectly measuring the resultent volumetric blood flow, characterized by porous member (11) having therein at least one aperture (16), by porous member (11) being placed on an air-impermeable base member (22, 18') having an opening therethrough in the area of aperture (16), and by the blood being movable in a manner to pass through aperture (16) while being prevented from flowing past porous member (11) along the sides thereof.

2. Assembly as in claim 1, characterized by the diameter of aperture (16) not being greater than 500 μm.

3. Assembly as in claim 2, characterized by the diameter of aperture (16) not being greater than 300 μm.

4. Assembly as in any one of claims 1 to 3, characterized by the diameter of the aperture not being smaller than 50 μm.

5. Assembly as in any one of claims 1 to 4, characterized by the diameter of aperture (16) being between 150 and 250 μm.

6. Assembly as in any one of claims 1 to 5, characterized by porous member (11) consisting of a filter material having a pore size smaller than 5 μm and greater than 0.01 μm.

7. Assembly as in any one of claims 1 to 6, characterized by porous member (11) having a plurality of apertures therethrough.

8. Assembly as in any one of claims 1 to 7, characterized by a capillary (9) provided on the upstream or downstream side of aperture (16), and by the capillary having an inner diameter between 50 and 500 μm.

9. Assembly as in claim 8, characterized by the length of capillary (9) being 0.5 to 5 cm.

10. Assembly as in any one of claims 1 to 9, characterized by porous member (11) having the shape of a disc or of an elongated tube.

11. Assembly as in any one of claims 1 to 10, characterized by porous member (11) consisting of a cellulose acetate material.

12. Assembly as in any one of claims 5 to 11, characterized by the assembly being designed as a disposable item and essentially comprising a tube (1') provided with a scale (17') and having in an end portion a bulge for receiving porous member (11), by capillary (9') being coupled to the bulge at the side thereof opposite tube (1') in the area of aperture (16) of porous member (1), by tube (1'), the bulge and capillary (9') being formed of a plastic material and being joined in the region of the bulge in a firm and sealed relationship following insertion of porous member (11) into the bulge.

13. A method of measuring bleeding time in vitro using an assembly as stated in any one of claims 1 to 12, characterized by the material of porous member (11) being impregnated prior to starting an examination with a solution aggregating blood platelets (12).

14. A method as in claim 13, characterized by using adenosine diphosphate for said solution.

15. A method as in one of claims 13 and 14, characterized by the material of porous member (11) having collagen dispersed therethrough.

16. A method as in any one of claims 13 to 15, characterized by growing biologic live cells on the surface of porous member (11) at least in the area of aperture (16) in a culture device prior to starting an examination.

17. A method as in any one of claims 13 to 16, characterized by measuring the resultant volumetric blood flow by detecting displacement of a liquid column (7) in a tube (1) as caused by positive or negative pressure.

18. A method as in any one of claims 13 to 16, characterized by determining the degree of occlusion of aperture (16) by measuring the difference of pressures existing directly on both sides of aperture (16).

## Revendications

1. Dispositif pour la mesure du temps de saignement in vitro, dans lequel le sang peut être acheminé sous pression à travers une pièce poreuse et dans lequel est prévu un dispositif de mesure directe ou indirecte du débit volumique résultant du sang, caractérisé en ce qu'au moins une ouverture (16) est prévue dans la pièce poreuse (11), en ce que la pièce poreuse (11) repose sur un support (22, 18') imperméable à l'air, présentant un orifice au voisinage de l'ouverture (16), et en ce que le sang est amené de manière à traverser l'ouverture (16) sans pouvoir parvenir sur les côtés de la pièce poreuse (11).

2. Dispositif selon la revendication 1, caractérisé en ce que le diamètre de l'ouverture (16) n'est pas supérieur à 500 μm.

3. Dispositif selon la revendication 2, caractérisé en ce que le diamètre de l'ouverture (16) n'est pas supérieur à 300 μm.

4. Dispositif selon l'une des revendications 1 à 3, caractérisé en ce que le diamètre de l'ouverture (16) n'est pas inférieur à 50 μm.

5. Dispositif selon l'une des revendications 1 à 4, caractérisé en ce que le diamètre de l'ouverture (16) est compris entre 150 et 200 μm.

6. Dispositif selon l'une des revendications 1 à 5, caractérisé en ce que la pièce poreuse (11) est constituée d'une matière filtrante dont la grosseur des pores est inférieure à 5 μm et supérieure à 0,01 μm.

7. Dispositif selon l'une des revendications 1 à 6, caractérisé en ce que plusieurs ouvertures sont prévues dans la pièce poreuse (11).

8. Dispositif selon l'une des revendications 1 à 7, caractérisé en ce qu'un tube capillaire (9) est raccordé devant et derrière l'ouverture (16) et en ce que le capillaire présente un diamètre intérieur compris entre 50 et 500 μm.

9. Dispositif selon la revendication 8, caractérisé en ce que la longueur du capillaire (9) est comprise entre 0,5 et 5 cm.

10. Dispositif selon l'une des revendications 1 à 9, caractérisé en ce que la pièce poreuse (11) présente la forme d'un disque ou d'un tube allongé.

11. Dispositif selon l'une des revendications 1 à 10, caractérisé en ce que la matière constituant la pièce poreuse (11) est de l'acétate de cellulose.

12. Dispositif selon l'une des revendications 5 à 11, caractérisé en ce qu'il est conçu pour être jeté et comprend essentiellement une canule (1') comportant une échelle (17') et présentant, à une extrémité, une incurvation recevant la pièce poreuse (11), en ce que le capillaire (9') est relié à l'incurvation, au voisinage de l'ouverture (16) de la pièce poreuse (11), du côté de l'incurvation opposé à celui de la canule (1'), en ce que la canule (1'), l'incurvation et le capillaire (9') sont formés en matière plastique et sont assemblés fermement et de façon étanche avec l'incurvation, après introduction de la pièce poreuse (11) dans l'incurvation.

13. Procédé de mesure du temps de saignement in vitro, à l'aide d'un dispositif selon l'une des revendications 1 à 12, caractérisé en ce que la matière constituant la pièce poreuse (11) est imprégnée, avant le début d'une analyse, d'une solution agglomérant les plaquettes de sang (12).

14. Procédé selon la revendication 13, caractérisé en ce qu'on utilise une solution de diphosphate d'adénosine.

15. Procédé selon l'une des revendications 13 ou 14, caractérisé en ce que la matière constituant la pièce poreuse (11) est imprégnée de collagène.

16. Procédé selon l'une des revendications 13 à 15, caractérisé en ce qu'avant le début d'une analyse, on fait croître, dans une installation pour cultures, des cellules biologiques vivantes sur la face supérieure de la pièce poreuse (11), au moins au voisinage de l'ouverture (16).

17. Procédé selon l'une des revendications 13 à 16, caractérisé en ce que, pour mesurer le débit volumique résultant du sang, on mesure dans un tube (1) le déplacement d'une colonne liquide (7) provoqué par une pression positive ou négative.

18. Procédé selon l'une des revendications 13 à 16, caractérisé en ce que, pour mesurer le degré d'obstruction de l'ouverture (16), on mesure directement la différence des pressions régnant de chaque côté de l'ouverture (16).

0 1 1 1 942

FIG. 1

FIG. 2

FIG. 3

1